(19) Europäisches Patentamt
European Patent Office
Office européen des brevets

(11) Veröffentlichungsnummer: **0 279 322**
**A2**

(12) **EUROPÄISCHE PATENTANMELDUNG**

(21) Anmeldenummer: **88101832.9**

(22) Anmeldetag: **09.02.88**

(51) Int. Cl.4: **C07C 45/46**, C07C 49/784, C07C 49/84, C07C 49/813

(30) Priorität: **16.02.87 DE 3704810**

(43) Veröffentlichungstag der Anmeldung:
**24.08.88 Patentblatt 88/34**

(84) Benannte Vertragsstaaten:
**AT BE CH DE ES FR GB GR IT LI LU NL SE**

(71) Anmelder: **BOEHRINGER INGELHEIM KG**
**Postfach 200**
**D-6507 Ingelheim am Rhein(DE)**

(84) **AT BE CH DE ES FR GB GR IT LI LU NL SE**

(71) Anmelder: **BOEHRINGER INGELHEIM INTERNATIONAL G.M.B.H.**
**Postfach 20**
**D-6507 Ingelheim am Rhein(DE)**

(84) **GB**

(72) Erfinder: **Müller, Manfred, Dr.-Ing.**
**Alsbacher Strasse 40**
**D-6101 Bickenbach(DE)**

(54) **Katalytische Acylierung von Aromaten in der Gasphase.**

(57) Die Erfindung betrifft ein Verfahren zur katalytischen Acylierung von Aromaten in der Gasphase in Gegenwart von Zeolithen.

EP 0 279 322 A2

Xerox Copy Centre

# Katalytische Acylierung von Aromaten in der Gasphase

Die Erfindung betrifft ein Verfahren zur katalytischen Acylierung von Aromaten in der Gasphase.

Die Friedel-Crafts-Acylierung ist ein klassisches Verfahren zur Herstellung von aromatischen Ketonen. Die Reaktion erfolgt im allgemeinen in einem organischen Lösungsmittel durch Umsetzung eines Benzolderivates mit einem Säurechlorid oder einem anderen reaktiven Säurederivat in Gegenwart einer Lewis-Säure, wie z.B. Aluminiumchlorid oder Zinkchlorid oder anderer Protonensäuren.

Nachteilig bei der Friedel-Crafts-Acylierung in der Flüssigphase ist die Verwendung großer Mengen organischer Lösungsmittel' einem hohen Überschuß an "Katalysator", z.B. $AlCl_3$, der im Verlauf der Reaktion aufgebraucht wird und die damit verbundene Salzbelastung des Abwassers bei der Produktaufarbeitung.

Gegenstand der Erfindung ist die Herstellung von gegebenenfalls substituierten Arylketonen durch die Umsetzung von gegebenenfalls substituierten Aromaten mit reaktiven Carbonsäurederivaten in der Gasphase in Gegenwart von Zeolithen.

Bevorzugt ist die Herstellung von Arylketonen der allgemeinen Formel I

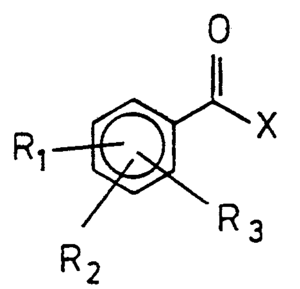

worin $R_1$, $R_2$ und $R_3$, die gleich oder verschieden sein können und Wasserstoff, Halogen, verzweigtes oder unverzweigtes Alkyl mit 1 bis 4 Kohlenstoffatomen, verzweigtes oder unverzweigtes Alkoxy mit 1 bis 4 Kohlenstoffatomen, eine gegebenenfalls substituierte Phenylgruppe,
und X einen Rest der allgemeinen Formel

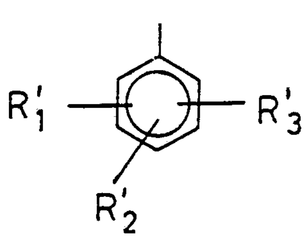

worin $R'_1$, $R'_2$, $R'_3$ dieselbe Bedeutung wie $R_1$, $R_2$ oder $R_3$ haben kann oder $R_3$ und $R'_3$ jeweils in o-Position zusammen eine Carbonylgruppe bilden können oder X eine verzweigte oder unverzweigte Alkylgruppe mit 1 bis 4 Kohlenstoffatomen, die gegebenenfalls durch Phenyl substituiert sein kann, bedeuten können. Als Halogen wird eines der Atome Fluor, Chlor, Brom oder Jod bezeichnet. Als Alkylgruppen werden - auch als Reste anderer Substituenten - Methyl, Ethyl, n-Propyl, sec. Propyl, n-Butyl, sec. Butyl und tert. Butylgruppen bezeichnet. Bevorzugte Alkylgruppen sind, soweit nichts anderes angegeben, Methyl, Ethyl, iso-Propyl, bevorzugte Alkoxygruppen Methoxy und Ethoxy. Weiterhin bevorzugt sind aromatische Reste, die mono-oder disubstituiert sind, wobei sterisch anspruchsvolle Reste nicht in ortho-Position zur Carbonylfunktion stehen sollten.

Ist einer der Substiuenten eine Phenylgruppe, so kann diese ebenfalls durch $R_1$, $R_2$ und $R_3$ substituiert sein wobei die Substituenten jedoch nicht wiederum Phenyl bedeuten können.

Verbindungen der allgemeinen Formel I erhält man durch Umsetzung von Verbindungen der allgemeinen Formel II

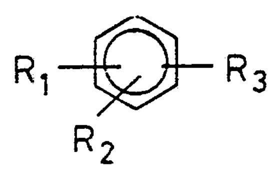

worin, $R_1$, $R_2$ und $R_3$ die zuvor genannte Bedeutung aufweisen, mit einem reaktiven Carbonsäurderivat der allgemeinen Formel

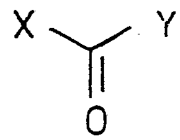

III

worin X einen Rest der allgemeinen Formel

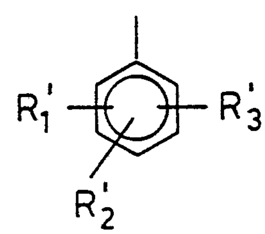

worin, $R'_1$, $R'_2$ und $R'_3$ die zuvor genannte Bedeutung aufweisen, oder X eine verzweigte oder unverzweigte Alkylgruppe mit 1 bis 4 Kohlenstoffatomen, die gebenenfalls durch Phenyl substituiert sein kann, und Y ein Halogenatom, bevorzugt Chlor oder Brom, eine Hydroxygruppe oder den Rest $O-\underset{\underset{O}{\|}}{C}-X$ oder $O-\underset{\underset{O}{\|}}{C}-$in der Bedeutung eines cyclischen Carbonsäureanhydrids bedeuten können, in der Gasphase in Gegenwart von Zeolithen in der H-Form.

Die Umsetzung erfolgt zweckmäßigerweise bei Temperaturen zwischen 200 und 500°C, bevorzugt zwischen 250 und 450°C.

Geeignete Zeolithe in der H-Form sind beispielsweise Mordenit, ZSM-5 und Faujasit.

Die Herstellung und Aktivierung der Zeolithe ist in der Fachliteratur hinreichend beschrieben und bedarf keiner weiteren Erläuterung. Einige Zeolithe, wie z.B. der Mordenit, sind als Handeisprodukte erhältlich.

Im allgemeinen erfolgt die Umsetzung der Komponenten II und III dadurch, daß die Ausgangsverbindungen verdampft und durch einen mit dem Zeolithen beschickten Reaktor geleitet werden. Wenn es erforderlich erscheint, kann zusätzlich ein inertes Trägergas, z.B. Stickstoff, zur Verdünnung der Reaktanden eingesetzt werden. Die Reaktionsbedingungen, wie z.B. Temperatur, Druck, Verweilzeit, Strömungsgeschwindigkeit, Mischungsverhältnis der Reaktanden und Packungsdichte des Zeolithen hängen von der Reaktivität der einzelnen Komponenten ab und können durch einfache Versuche ermittelt werden.

Die Aufarbeitung der nach dem erfindungsgemäßen Verfahren hergestellten Arylketone ist unproblematisch. Der aus dem Reaktor austretende Gasstrom wird kondensiert, wobei die entstandenen Arylketone aus den flüssigen, nicht umgesetzten Ausgangsverbindungen im allgemeinen auskristallisieren. Nach der Trennung können die Ausgangsverbindungen wieder in den Reaktor zurückgeführt werden. Die Produkte können, wenn es notwendig erscheint, durch Kristallisation oder Destillation weiter gereinigt werden.

Bevorzugte Verbindungen der allgemeinen Formel II sind 1,2-Dimethyloxybenzol, Anisol, Benzol; bevorzugte reaktive Säurederivate der allgemeinen Formel III sind Benzoylchlorid, p-Chlorbenzoylchlorid, Acetylchlorid, Propionylchlorid, Acetanhydrid, Maleinsäureanhydrid, Pyromellitsäuredianhydrid, Phthalsäureanhydrid.

Gegenüber der Friedel-Crafts-Acylierung in der Flüssigphase bietet die kontinuierliche Acylierung in der Gasphase an Zeolithen folgende Vorteile:

1. Hohe Selektivität der Reaktion, hohe Aktivität und gute Beständigkeit des Katalysators.

2. Problemlosere umweltschonendere Aufarbeitung des Reaktionsgemisches (keine Zersetzung wie z.B. bei $AlCl_3$).

3. Wiederverwendbarkeit des Katalysators.

4. Geringere Herstellkosten.

Das nachfolgende Beispiel soll die Erfindung erläutern, ohne sie jedoch zu beschränken.

Beispiel: 4-Chlor-3',4'-dimethoxybenzophenon

Ausgangsverbindungen: Veratrol, p-Chlorbenzoylchlorid
Reaktor: Rohr 1 cm ∅, 1 m lang gefüllt mit 40 ml Mordenit (H-Form) gepreßt zu Pellets von 1,5 mm Kantenlänge und 5 mm Durchmesser
Trägergas: Stickstoff, 430 ltr/h

25,4 g/h Veratrol und 32,2 g/h p-Chlorbenzoylchlorid werden bei Normaldruck und 250°C verdampft und bei einer Salzbadtemperatur von ca. 420°C über den Zeolithen (Mordenit) geleitet. Das austretende Gas wird gequencht, wobei das Produkt nach kurzer Zeit auskristallisiert. Der gesammelte Ofenaustrag wird nach ca. 3 h Laufzeit von der flüssigen Phase (Ausgangsprodukte) getrennt und in bekannter Weise durch Umkristallisation aus Isopropanol gereinigt. Man isoliert 32,3 g 4-Chlor-3',4'-dimethoxybenzophenon; dies entspricht einer Ausbeute von 22 % (ca. 37 % bez. auf Umsatz).

**Ansprüche**

1) Verfahren zur Herstellung von Arylketonen ausgehend von gegebenenfalls substituierten Aromaten durch Umsetzung mit reaktiven Carbonsäurederivaten, dadurch gekennzeichnet, daß man die Umsetzung in der Gasphase in Gegenwart von Zeolithen in der H-Form durchführt.

2) Verfahren zur Herstellung von Arylketonen der allgemeinen Formel I

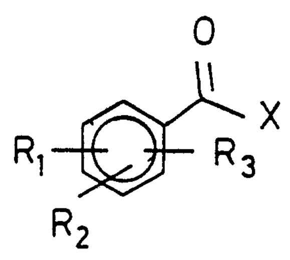

worin $R_1$, $R_2$ und $R_3$, die gleich oder verschieden sein können und Wasserstoff, Halogen, verzweigtes oder unverzweigtes Alkyl mit 1 bis 4 Kohlenstoffatomen, verzweigtes oder unverzweigtes Alkoxy mit 1 bis 4 Kohlenstoffatomen, eine gegebenenfalls substituierte Phenylgruppe,
und X einen Rest der allgemeinen Formel

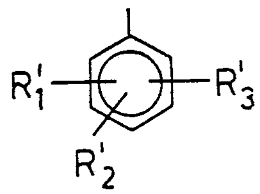

worin $R'_1$, $R'_2$, $R'_3$ dieselbe Bedeutung wie $R_1$, $R_2$ oder $R_3$ haben kann oder $R_3$ und $R'_3$ jeweils in o-Position zusammen eine Carbonylgruppe bilden können
oder X eine verzweigte oder unverzweigte Alkylgruppe mit 1 bis 4 Kohlenstoffatomen, die gegebenenfalls durch Phenyl substituiert sein kann, nach Anspruch 1, dadurch gekennzeichnet, daß man eine Verbindung der allgemeinen Formel II

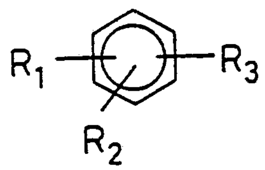

worin, $R_1$, $R_2$ und $R_3$ die zuvor genannte Bedeutung aufweisen, mit einem reaktiven Carbonsäurderivat der allgemeinen Formel

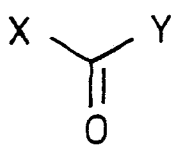

III

worin X einen Rest der allgemeinen Formel

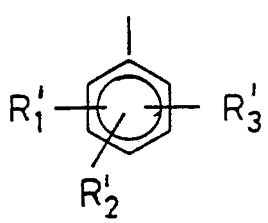

worin, $R'_1$, $R'_2$ und $R'_3$ die zuvor genannte Bedeutung aufweisen oder
X eine verzweigte oder unverzweigte Alkylgruppe mit 1 bis 4 Kohlenstoffatomen, die gebenenfalls durch Phenyl substituiert sein kann, und Y ein Halogenatom, bevorzugt Chlor oder Brom, eine Hydroxygruppe oder den Rest
$O-\underset{\underset{O}{\|}}{C}-X$ oder $O-\underset{\underset{O}{\|}}{C}-$in der Bedeutung
eines cyclischen Carbonsäureanhydrids bedeuten können, in der Gasphase in Gegenwart von Zeolithen in der H-Form, umsetzt.

3) Verfahren nach Anspruch 1 oder 2, dadurch gekennzeichnet, daß der Aromat aus der Gruppe Benzol, Anisol oder 1,2-Dimethyloxybenzol, das Carbonsäurederivat aus der Gruppe Benzoylchlorid, p-Chlorbenzoylchlorid, Acetylchlorid, Propionylchlorid, Acetanhydrid, Maleinsäureanhydrid, Pyromellitsäuredianhydrid ausgewählt wird.

4) Verfahren nach einem der Ansprüche 1 bis 3, dadurch gekennzeichnet, daß die Reaktion bei einer Temperatur zwischen 200 und 500°C durchgeführt wird.

5) Verfahren nach einem der Ansprüche 1 bis 4, dadurch gekennzeichnet, daß der Zeolith aus der Gruppe Mordernit, ZSM-5 oder Faujasit ausgewählt wird.